(19) Europäisches Patentamt
European Patent Office
Office européen des brevets

(11) Numéro de publication: 0 206 950 B1

(12) # FASCICULE DE BREVET EUROPEEN

(45) Date de publication du fascicule du brevet: 22.08.90

(51) Int. Cl.[5]: C07C 29/40, C07C 33/46, C07C 43/23, C07C 205/35, C07D 213/30, C07D 307/42, C07C 31/40, C07C 31/44, C07C 33/48

(21) Numéro de dépôt: 86420129.8

(22) Date de dépôt: 16.05.86

(54) **Procédé de préparation d'alcools perfluoroalkylés.**

(30) Priorité: 22.05.85 FR 8507696

(43) Date de publication de la demande: 30.12.86 Bulletin 86/52

(45) Mention de la délivrance du brevet: 22.08.90 Bulletin 90/34

(84) Etats contractants désignés: AT BE CH DE FR GB IT LI LU NL SE

(56) Documents cités: US-A- 4 484 993

(73) Titulaire: RHONE-POULENC CHIMIE, 25, quai Paul Doumer, F-92408 Courbevoie Cédex(FR)

(72) Inventeur: Francese, Catherine, 111, rue de Chevilly, F-92240 l'Hay les Roses(FR)
Inventeur: Tordeux, Marc, 1, rue de Seignelay, F-92330 Sceaux(FR)
Inventeur: Wakselman, Claude, 5, rue Chanez, F-75016 Paris(FR)

(74) Mandataire: Le Pennec, Magali et al, RHONE-POULENC INTERSERVICES Service Brevets Chimie 25, Quai Paul Doumer, F-92408 Courbevoie Cédex(FR)

EP 0 206 950 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de préparation d'alcools perfluoroalkylés. Elle concerne plus particulièrement un procédé de perfluoroalkylation de composés carbonylés.

Il est connu, d'après KITAZUME et ISHIKAWA (Chemistry Letters, 1981, p. 1679-1680), de fixer des iodures de trifluorométhyle sur des composés carbonylés dans un solvant aprotique polaire en présence de zinc et d'ultrasons. Les auteurs affirment dans ce texte qu'en l'absence d'ultrasons, aucune réaction ne se produit. Les ultrasons n'étant pas utilisables pour des procédés à grande échelle, cette technique ne peut donc être retenue au niveau industriel. Des essais selon le même procédé ont aussi été effectués avec des iodures de perflucroéthyle et propyle SOLLADIE-CAVALLO, FARKHANI, FRITZ, LAZRAK, SUFFERT, Tétrahedron letters, 25, 4117-4120, 1984).

Il est encore connu, d'après O'REILLY, MARUTA, ISHIKAWA, (Chemistry Letters, 517-520, 1984) de perfluoralkyler des aldéhydes par réaction d'iodures de perfluoralkyle avec différents aldéhydes en présence de catalyseurs choisis parmi les complexes triphénylphosphine-palladium ou -nickel. La réaction est utilisable industriellement mais le prix du catalyseur rend, d'un point de vue économique, cette réaction difficile à mettre en oeuvre.

La présente invention a pour objet un procédé utilisable industriellemnt. Elle concerne un procédé de perfluoralkylation de composés carbonylés caractérisé en ce que l'on met en présence un dérivé carbonylé, un iodure ou un bromure de perfluoroalkyle et du zinc dans un solvant aprotique polaire et/ou une pyridine.

Ce procédé présente l'avantage, par rapport à tous les procédés connus dans l'art antérieur, de n'utiliser ni catalyseurs ni appareillage impossible à mettre en oeuvre industriellement.

Les iodures ou bromures de perfluoroalkyle sont choisis de préférence parmi le bromure de trifluorométhyle et les iodures de perfluoralkyle dont la chaîne alkyle perfluorée contient de 2 à 12 atomes de carbone. Cette préférence n'est pas due à une différence de réactivité des différents composés mais uniquement à une différence de coût. En effet, le bromotrifluorométhane est beaucoup moins cher que l'iodotrifluorométhane et inversement les iodures de perfluoroéthyle, perfluorobutyle... sont beaucoup moins chers que leurs analogues bromés.

Le dérivé carbonylé répond de préférence à la formule générale (I) suivante :

$$R - \underset{\underset{O}{\|}}{C} - R' \qquad (I)$$

dans laquelle:
R et R' identiques ou différents sont choisis parmi l'hydrogène, les groupes alkyles ou alkényles linéaires ou ramifiés contenant 1 à 20 atomes de carbone et de préférence 1 à 12 atomes de carbone, alicycliques, aromatiques monocycliques, polycycliques ou hétérocycliques éventuellement substitués par un radical choisi parmi les éléments: hydroxyle, halogène, alkyle, alkényle, alcoxy, alkylthio, dialkylamino, nitrile, ester, amide, aryl, aryloxy, arylthio, fluoroalkyl, fluoroalcoxy, fluoroalkylthio.

Parmi les composés de formule (I) on peut citer mais non limitativement les benzaldéhydes tels que les méthylbenzaldéhydes, les méthoxybenzaldéhydes, les fluoro, bromo, chloro benzaldéhydes, les cyanobenzaldéhydes, les diméthylaminobenzaldéhydes, les naphtylcarboxaldéhydes, les anthrylcarboxaldéhydes, les pyridylcarboxaldéhydes, le furfural, le formaldéhyde, l'acétaldéhyde, le propanal, le butanal, le cyclohexylcarboxaldéhyde, le cinnamaldéhyde, les cyclohexanones, l'acétophénone, les pyruvates de méthyle ou d'éthyle.

Pour une mise en oeuvre avantageuse de l'invention, on utilise un solvant aprotique polaire et/ou une pyridine.

Les solvants aprotiques polaires utilisables dans le procédé de l'invention sont choisis non limitativement parmi le diméthylformamide, le diméthylsulfoxide, le diméthylacétamide, la N-méthylpyrolidone, l'hexaméthylphosphoramide.

On préfère utiliser le diméthylformamide et/ou une pyridine.

Parmi les pyridines on peut prendre l'une quelconque d'entre elles substituées ou non par un ou plusieurs groupes alkyles telles que la méthyl ou diméthylpyridine, mais on préfère utiliser la pyridine non substituée.

La pyridine permet de réduire considérablement le temps d'induction de la réaction.

Selon une mise en oeuvre avantageuse de l'invention, on utilise de préférence un rapport molaire du zinc au dérivé carbonylé supérieur ou égal à 1 et inférieur à 2 et un rapport molaire de l'iodure ou du bromure de perfluoroalkyle au dérivé carbonylé supérieur ou égal à 1. Si l'halogénure de perfluoralkyle est mis en excès et que l'halogénure utilisé est le bromure de trifluorométhyle, celui-ci sera aisément recyclé car il se présente sous forme gazeuse.

Pour une meilleure mise en oeuvre de la réaction, on préfère maintenir une température inférieure à 115°C et encore plus préférentiellement une température comprise entre - 20°C et 90°C.

La température sera adaptée par l'homme de l'art au produit à perhaloalkyler, ainsi, par exemple, on utilisera une température d'environ 0°C pour les aldéhydes linéaires, une température d'environ 90°C pour le formaldéhyde et la température ambiante pour l'ensemble des autres composés.

On opère de préférence en l'absence d'oxygène.

La pression est de préférence supérieure ou égale à la pression atmosphérique et notamment comprise entre 1 et 10 bars. Parmi les produits obtenus selon le procédé de l'invention, on peut citer: le phényl-1 trifluoro-2,2,2 éthanol, les méthyl, méthoxy, fluoro, chloro, bromo, cyano, diméthylamino phényl-1 trifluoro-2,2,2 éthanols, le phényl-1 pentafluoro-2,2,3,3,3 propanol, le (naphtyl-1)-1 trifluoro-2,2,2 éthanol, l'(anthryl-9)-1 trifluoro-2,2,2 éthanol, les pyridyl-1 trifluoro-2,2,2 éthanols, le (furfuryl-2)-1 trifluoro-2,2,2 éthanol, le benzoate de trifluoro-2,2,2 éthyle, le trifluoro-1,1,1 butanol-2, le cyclohexyl-1 trifluoro-2,2,2 éthanol, le phényl-4 trifluoro-1,1,1 butène-3 ol-2, le n-perfluorohexyl phénylméthanol, le trifluoro-1,1,1 phényl-2 propanol-2, le trifluoromethyl-1 cyclohexanol, les hydroxy-2 trifluorométhyl-2 propionates de méthyle ou d'éthyle.

Les produits obtenus par le procédé selon l'invention sont utilisés comme intermédiaires de synthèse dans l'industrie pharmaceutique et phytosanitaire, dans la préparation de polymères (HITO macromolecule 1982, 15, 915), pour la préparation de cristaux liquides et pour la séparation d'isomères optiques (J. Organic Chemistry 42, 384, 1370, 1977).

L'invention sera plus complètement décrite à l'aide des exemples suivants qui ne doivent pas être considérés comme limitatifs de l'invention.

EXEMPLES 1 A 9 - BENZALDEHYDES

Dans un flacon en verre épais, on met 25 ml de pyridine, 10ml de benzaldéhyde (0,0985 mole) et 6,5g de zinc en poudre (0,1 mole).

Le flacon est placé dans un appareil de Parr. On y fait le vide puis on introduit du bromotrifluorométhane jusqu'à une pression de 2,6 bars. On agite le flacon pendant le temps de la réaction.

La réaction débute environ 5 minutes plus tard et est exothermique.

La pression de bromotrifluorométhane est maintenue entre 3 et 1,5 bars pendant la réaction qui dure environ une heure.

La quantité totale absorbée est de 30g (0,2 mole).

On filtre puis on hydrolyse par 50ml d'acide chlorhydrique à 10% glacé en agitant 30 minutes.

Après extraction à l'éther et lavage à l'eau, le benzaldéhyde non réagi est précipité par l'hydrogénosulfite de sodium.

Pour cela, on laisse agiter une heure la phase éthérée additionnée du réactif bisulfitique, préparé avec 120ml d'une solution d'hydrogénosulfite de sodium à 38% et 30ml de méthanol, puis on filtre et on extrait à l'éther.

Après séchage sur sulfate de magnésium et évaporation du solvant, le phényl-1trifluoro-2,2,2 éthanol est distillé sous vide :Eb=75°/12mmHg. On obtient 9g (52%) ($Eb_{litt}$=84-87°/14mmHg). RMN$^{19}$F($CFCl_3$ext) : -77,7ppm (d, $J_{F-H}$=7,1Hz) RMN$^1$H (TMS int.) : 7,5ppm (Ar-H) ; 5ppm (q, CH) ; 3,5ppm (OH)

Les résultats pour les benzaldéhydes substitués en position 4 sont résumés dans le tableau suivant :

| Ex. | Substituant | Prise d'essai | Masse obtenue | Eb |
|---|---|---|---|---|
| 1 | H | 10ml(0,0985 mole) | 9g (52%) | 75°/12mm |
| 2 | $CH_3$ | 11ml(0,0934 mole) | 9,4g (53%) | (a) 86-90°/10mm |
| 3 | $CH_3O$ | 12ml(0,0987 mole) | 9,5g (47%) | 122°/13mm |
| 4 | F | 10ml(0,0933 mole) | 11g (60%) | 74-76°/15mm |
| 5 | Cl | 13g(0,0929 mole) | 9g (46%) | (b) 90-95°/12mm |
| 6 | Br | 10g(0,0541 mole) | 3g (22%) | 88-90°/0,1mm |
| 7 | CN | 12g(0,0916 mole) | 8g (43%) | PF = 89°C |
| 8 | $N(CH_3)_2$ | 14g (0,094 mole) | < 10 % | |
| 9 | $NO_2$ | 13g (0,0861 mole) | 0 | |

(a) $Eb_{litt}$ = 102°/15mm Hg

(b) $Eb_{litt}$ = 80°/4mm Hg

EXEMPLES 10 A 16 - BENZALDEHYDE ET SOLVANTS

Dans un flacon en verre épais, on met 25ml de solvant, 10ml de benzaldéhyde (0,0985 mole) et 6,5g de zinc (0,1 mole).

Le flacon est placé dans un appareil de Parr. On y fait le vide puis on introduit du bromotrifluorométhane jusqu'à une pression de 2,6 bars; celle-ci étant maintenue entre 3 et 1,6 bars pendant la réaction. On agite pendant tout le temps de la réaction.

On filtre puis on hydrolyse par 50ml d'acide chlorhydrique à 10% glacé en agitant 30 minutes.

Après extraction à l'éther et lavage à l'eau, le benzaldéhyde non réagi est précipité par l'hydrogénosulfite de sodium, puis on filtre et on extrait à l'éther.

Après sèchage sur sulfate de magnésium et évaporation du solvant, on obtient le phényl-1 trifluoro-2,2,2 éthanol.

| EXEMPLES | SOLVANT | RENDEMENT |
|---|---|---|
| 10 | Diméthylacétamide | 10% |
| 11 | Diméthylformamide | 25% |
| 12 | Diméthylsulfoxide | 30% |
| 13 | Hexaméthylphosphoramide | 10% |
| 14 | N-méthylpyrolidone | 5% |
| 15 | 2-picoline | 20% |
| 16 | Pyridine+diméthylsulfoxide<br>5ml 25ml | 35% |

EXEMPLE 17 - BENZALDEHYDE $CF_3I$

Dans un flacon en verre épais, on met 25ml de pyridine, 10ml de benzaldéhyde (0,0985 mole) et 6,5g de zinc en poudre (0,1 mole).

Le flacon est placé dans un appareil de Parr. On y fait le vide puis on introduit 24g (0,122 mole) de iodotrifluorométhane.

La réaction débute environ 5 minutes plus tard et est exothermique.

On procède ensuite comme à l'exemple 1.

Après extraction à l'éther, lavage à l'eau, séchage sur sulfate de magnésium et évaporation du solvant, le phényl-1 trifluoro-2,2,2 éthanol est distillé sous vide; Eb=75°/12mm Hg. On obtient 5,8g (33,5%)

EXEMPLE 18 - BENZALDEHYDE $C_2F_5I$

Dans un flacon en verre épais, on met 25ml de pyridine, 10ml de benzaldéhyde (0,0985 mole) et 6,5g de zinc en poudre (0,1 mole).

Le flacon est placé dans un appareil de Parr. On y fait le vide, puis on introduit 32g (0,130 mole) de iodopentafluoroéthane.

La réaction débute environ 5 minutes plus tard et est exothermique.

On procède ensuite comme à l'example 1.

Après extraction à l'éther, lavage à l'eau, sèchage sur sulfate de magnésium et évaporation du solvant, le phényl-1 pentafluoro-2,2,3,3,3 propanol est distillé sous vide : Eb = 72-75°/10mm Hg. On obtient 6g (27%) ($Eb_{litt}$=84-85°/10mm Hg). RMN$^{19}$F ($CFCl_3$ ext) : -81ppm (s, $CF_3$) ;
- 127,8ppm (d.d,$CF_2$,$J_{F-F}$=274,5 Hz,$J_{F-H}$=15 Hz);
-121,7ppm (d.d, $CF_2$,$J_{F-H}$=9,4 Hz) ;
RMN$^1$H (TMS int.) : 7,4ppm (Ar-H) ; 5,1ppm (d.d, CH) ; 3ppm (OH).

EXEMPLE 19 - NAPHTYL-1 CARBOXALDEHYDE

Dans un flacon en verre épais, on met 25ml de pyridine, 15ml de naphtyl-1 carboxaldéhyde (0,111 mole) et 7g de zinc en poudre (0,11 mole).

On procède ensuite comme à l'exemple 1.

Après extraction à l'éther, lavage à l'eau, sèchage sur sulfate de magnésium et évaporation du solvant, le (naphtyl-1)-1 trifluoro-2,2,2 éthanol est distillé sous vide : Eb = 116-118°/1mmHg. On obtient 3,6g (14%).
RMN$^{19}$F($CFCl_3$ ext) : - 66,7 ppm (d, $J_{F-H}$= Hz) ;
RMN$^1$H (TMS int.) : 8,2 - 7,3ppm (Ar-H) ; 5,8ppm (q, CH);3,3ppm(OH).

EXEMPLE 20 - ANTHRYL-9 CARBOXALDEHYDE

Dans un flacon en verre épais, on met 25 ml de pyridine, 10 g d'anthryl-9 carboxaldéhyde (0,0485 mole) et 6,5g de zinc en poudre (0,1 mole).

On procède ensuite comme à l'exemple 1.

Après extraction à l'éther, lavage à l'eau, séchage sur sulfate de magnésium et évaporation du solvant, on obtient 5g (37%) d'(anthryl-9)-1 trifluoro-2,2,2 éthanol qui sont ensuite sublimés.
PF=130-131°C ($PF_{litt.}$=142-144°C)
RMN$^{19}$F ($CFCl_3$ ext) : - 74,7ppm (d, $J_{F-H}$= 8,5 Hz ;
RMN$^1$H ($CD_3CN$, TMS int.) : 8,8 - 7,4 ppm (Ar-H) ; 6,8ppm (q, CH) ;
3ppm (OH).

EXEMPLE 21 - PYRIDYL-2 CARBOXALDEHYDE

Dans un flacon en verre épais, on met 25 ml de pyridine, 10ml de pyridyl-2 carboxaldéhyde (0,105 mole) et 6,5 de zinc en poudre (0,1 mole).

On procède ensuite comme à l'exemple 1.

Après extraction au dichlorométhane, lavage à l'eau, séchage sur sulfate de magnésium et évaporation du solvant, on obtient 12g (65%) de (pyridyl-2)-1 trifluoro-2,2,2 éthanol qui sont ensuite sublimés. PF = 45,5°C.
RMN$^{19}$F ($CFCl_3$ ext) : - 77,7ppm (d, $J_{F-H}$ = 6,6Hz)
RMN$^1$H (TMS int.) : 9-7,3ppm (Ar-H) ; 6,1ppm (OH) ; 5,2ppm (q, CH).

EXEMPLE 22 - FURFURAL

Dans un flacon en verre épais, on met 25ml de pyridine, 10ml de furfural (0,121 mole) et 8g de zinc en poudre (0,12 mole).

Le flacon est placé dans un appareil de Parr. On y fait le vide puis on introduit du bromotrifluorométhane jusqu'à une pression de 2,6 bars.

On procède ensuite comme à l'exemple 1.

Après extraction à l'éther, lavage à l'eau, séchage sur sulfate de magnésium et évaporation du solvant, le (furfuryl-2)-1 trifluoro-2,2,2 éthanol est distillé sous vide : Eb = 60°/12mm Hg. On obtient 5,2g (26%).

RMN$^{19}$F ($CFCl_3$ext) : - 77,7ppm (d, $J_{F-H}$ = 7,1Hz)
RMN$^1$H(TMS int.) : 7,4 - 6,2ppm (Ar-H);4,9ppm (q, CH);3,5ppm (OH).

EXEMPLE 23 - FORMALDEHYDE

Dans un flacon en verre épais, on met 50ml de pyridine, 5g de formaldéhyde (0,167 mole) et 11g de zinc (0,169 mole).

Le flacon est placé dans un appareil de Parr. On y fait le vide, on chauffe à 90°C, puis on introduit du bromotrifluorométhane jusqu'à une pression de 2,6 bars. On agite.

La réaction débute environ 30 minutes plus tard et la pression de bromotrifluorométhane est maintenue entre 3 et 1,5 bars pendant la réaction qui dure environ 2 heures.

On filtre puis on additionne 10ml de chlorure de benzoyle. La réaction est exothermique. On hydrolyse par 10 ml d'acide chlorhydrique à 10% glacé et on extrait à l'éther. Après évaporation du solvant, le benzoate de trifluoro-2,2,2 éthyle est purifié par chromatographie sur couche mince. On obtient 3,5g (10%).

Benzoate de trifluoro-2,2,2 éthyle:
RMN$^{19}$F $CFCl_3$ ext.) : - 73ppm (t,$J_{F-H}$ = 9,4 Hz) ;
RMN$^1$H (TMS int.) : 8,9-7,3ppm (Ar-H) ;
4,7ppm (q, $CH_2$)

EXEMPLE 24 - BUTANAL

Dans un flacon en verre épais, on met 25ml de pyridine, 10ml de butanal (0,113 mole) et 8 g de zinc (0,123 mole).

Le flacon est placé dans un appareil de Parr. On y fait le vide, on refroidit à 0°C, puis on introduit du bromotrifluorométhane jusqu'a une pression de 2,6 bars.

La réaction débute environ 5 minutes plus tard et est exothermique. La pression de bromotrifluorométhane est maintenue entre 3 et 1,5 bars pendant la réaction qui dure environ une demi-heure. On agite le flacon pendant toute la durée de la réaction.

On filtre puis on hydrolyse par 15ml d'acide sulfurique à 20% pendant 8 heures.

Après extraction à l'éther, lavage à l'eau, séchage sur sulfate de magnésium et évaporation du solvant, le trifluoro-1,1,1 pentanol-2 est distillé : Eb = 92-94°/760mmHg. On obtient 4,8g(30%).
RMN$^{19}$F ($CFCl_3$ ext.) : - 79,5ppm (d, $J_{F-H}$ = 7,5 Hz);
RMN$^1$H (TMS int.) : 3,9 ppm (m, CH) ; 3,2ppm (OH)
2,7-1,3ppm(m,$CH_2$-$CH_2$) ;
1,2-0,8ppm (m, $CH_3$)

EXEMPLE 25 - CYCLOHEXYLCARBOXALDEHYDE

Dans un flacon en verre épais, on met 25ml de pyridine, 10ml de cyclohexylcarboxaldéhyde (0,0827 mole) et 6,5g de zinc en poudre (0,1 mole).

On procède ensuite comme à l'exemple 1.

Après extraction à l'éther, lavage à l'eau, sèchage sur sulfate de magnésium et évaporation du solvant, le cyclohexyl-1 trifluoro-2,2,2 éthanol est distillé sous vide : Eb = 60-64°/12mm Hg. On obtient 4,5g (30%).
RMN$^{19}$F ($CFCl_3$ ext) : - 75,5ppm (d, $J_{F-H}$ = 7,7Hz) ;
RMN$^1$H (TMS int.) : 4,1-3,4 (m, CH) ; 2,5ppm (OH) ;
2,2-0,9ppm (m, 10 H).

EXEMPLE 26 - CINNAMALDEHYDE

Dans un flacon en verre épais, on met 25ml de pyridine, 12ml de cinnamaldéhyde (0,0955 mole) et 6,5g de zinc en poudre (0,1 mole).

On procède ensuite commme à l'exemple 1.

Après extraction à l'éther, lavage à l'eau, sèchage sur sulfate de magnésium et évaporation du solvant, le phényl-4 trifluoro-1,1,1 butène-3-ol-2 est distillé sous vide : Eb = 98-102°/0,5mmHg. On obtient 10,5g (55%) ($Eb_{litt}$ = 86-88°/5mmHg). RMN$^{19}$F ($CFCl_3$ ext) : - 77,7ppm (d, $J_{F-H}$ = 6,6Hz) ;
RMN$^1$H (TMS int.) : 7,4ppm (Ar-H) ; 6,9ppm (d, CH, $J_{trans}$ = 16Hz)
6,2ppm (d.d, CH, $J_{trans}$ = 16Hz, $^3J_{H,\ H}$=6Hz);
4,7ppm (q, CH) ; 3,6ppm (OH)

EXEMPLE 27 - BENZALDEHYDE - $C_6F_{13}I$

Dans un ballon on met 10ml de pyridine, 2ml de benzaldéhyde (0,0197 mole) et 2 g de zinc en poudre (0,03 mole). On purge à l'argon et on additionne ensuite 10g de iodo-1 perfluoro n-hexane (0,022 mole) en

agitant. La réaction est exothermique. On filtre puis on hydrolyse par 10ml d'acide chlorhydrique à 10% glacé en agitant 30 minutes.

Après extraction à l'éther et lavage à l'eau, le benzaldéhyde non réagi est précipité par l'hydrogénosulfite de sodium. Pour cela, on laisse agiter une heure la phase éthérée additionnée d'hydrogénosulfite de sodium à 38% et 30ml de méthanol puis on filtre et on extrait à l'éther.

Après sèchage sur sulfate de magnésium et évaporation du solvant, on obtient 4g (48%) de n-perfluorohexylphénylméthanol qui sont ensuite sublimés. PF = 47°C ($PF_{litt}$ = 49-51°C).

RMN$^{19}$F ($CFCl_3$ ext.) - 79,7ppm (t.t, $CF_3$) ;
-115 et -125ppm (d.d, $CF_2$, $J_{F-F}$ = 273 Hz) ;
- 118 - 122ppm et - 124-125ppm (m, 8 F).

RMN$^1$H (TMS int.) : 7,5ppm (Ar-H) ;
5,4-5,2 ppm (d. d, CH, $^3J_{F-H}$ = 17 Hz, $^3J_{F-H}$=8Hz)
3,8ppm (OH)

EXEMPLE 28 - ACETOPHENONE

Dans un flacon en verre épais, on met 25ml de pyridine, 10g d'acétophénone (0,0833 mole) et 6,5g de zinc en poudre (0,1 mole).

Le flacon est placé dans un appareil de Parr. On y fait le vide puis on introduit du bromotrifluorométhane jusqu'à une pression de 2,6 bars ; celle-ci étant maintenue entre 3 et 1,5 bars pendant la réaction qui dure environ 3 heurs. On agite pendant toute la réaction.

On filtre puis on hydrolyse par 50ml d'acide chlorhydrique à 10% glacé en agitant 30 minutes.

Après extraction à l'éther, lavage à l'eau, sèchage sur sulfate de magnésium et évaporation du solvant, le trifluoro-1,1,1 phényl-2 propanol-2 est purifié par chromatographie en phase gazeuse (colonne 10% dinonylphtalate sur chromosorb WDMCS-60/80 mesh) à 145°C.
RMN$^{19F}$ ($CFCl_3$ ext.) : - 80ppm (s, $CF_3$)
RMN$^1$H (TMS int.) : 6,8-6,3ppm (Ar-H)
1,99ppm (OH) ; 0,9ppm (s, $CH_3$)

EXEMPLE 29 - CYCLOHEXANONE

Dans un flacon en verre épais, on met 25ml de pyridine, 10g de cyclohexanone (0,102 mole) et 7 g de zinc en poudre (0,108 mole).

Le flacon est placé dans un appareil de Parr. On y fait le vide puis on introduit du bromotrifluorométhane jusqu'à une pression de 2,6 bars ; celle-ci étant maintenue entre 3 et 1,5 bars pendant la réaction qui dure environ 3 heures. On agite pendant toute la durée de la réaction.

On filtre puis on hydrolyse par 50ml d'acide chlorhydrique à 10% glacé en agitant 30 minutes.

Après extraction à l'éther, lavage à l'eau, sèchage sur sulfate de magnésium et évaporation du solvant, le trifluorométhyl-1 cyclohexanol est purifié par chromatographie en phase gazeuse (colonne : 10% dinonylphtalate sur chromosorb WDMCS 60/80 mesh) à 120°C. On obtient 3,4g (20%).
RMN$^{19}$F ($CFCl_3$ ext.) : - 83ppm (s, $CF_3$).

EXEMPLE 30 - PYRUVATE D'ETHYLE

Dans un flacon en verre épais, on met 25ml de pyridine, 10ml de pyruvate d'éthyle (0,0914 mole) et 6,5g de zinc en poudre (0,1 mole).

Le flacon est placé dans un appareil de Parr. On y fait le vide puis on introduit du bromotrifluorométhane jusqu'à une pression de 2,6 bars. On agite pendant toute la durée de la réaction.

La réaction débute environ 5 minutes plus tard et est exothermique. La pression de bromotrifluorométhane est maintenue entre 3 et 1,5 bars pendant la réaction qui dure environ une demi-heure.

On filtre, puis on hydrolyse par 30ml d'acide chlorhydrique à 10% glacé.

Après extraction à l'éther, lavage à l'eau, sèchage sur sulfate de magnésium et évaporation du solvant, l'hydroxy-2 trifluorométhyl-2 propionate d'éthyle est distillé sous vide. Eb = 60°/65m Hg. On obtient 6g (35%). ($Eb_{litt}$ = 140-142°/760mmHg).
RMN$^{19}$F ($CFCl_3$ ext.) : - 79,7ppm (s, $CF_3$).
RMN$^1$H (TMS int.) : 4,6ppm (q, $CH_2$, $^3J_{H-H}$ = 7Hz) ;
4,2ppm (OH) ; 1,7ppm (s, $CH_3$) ;
1,5ppm (t, $CH_3$).

**Revendications**

1. Procédé de perfluoroalkylation de composés carbonylés caractérisé en ce que l'on met en présence un dérivé carbonylé, un iodure ou un bromure de perfluoroalkyle et du zinc dans un solvant aprotique polaire et/ou une pyridine en l'absence d'ultrasons.

2. Procédé selon la revendication 1 caractérisé en ce que le bromure de perfluoroalkyle est le bromotrifluorométhane.

3. Procédé selon la revendication 1 caractérisé en ce que l'iodure de perfluoroalkyle est choisi parmi les iodures de perfluoroalkyle dont la chaîne alkyle perfluorée contient de 2 à 12 atomes de carbone.

4. Procédé selon la revendication 1 caractérisé en ce que le dérivé carbonylé répond à la formule générale (I) :

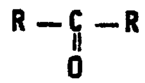

dans laquelle
R et R' identiques ou différents son choisis parmi l'hydrogène, les groupes alkyles ou alkényles linéaires ou ramifiés, contenant 1 à 20 atomes de carbone, alicycliques, aromatiques monocycliques, polycycliques ou hétérocycliques éventuellement substitués par un radical choisi parmi les éléments: hydroxyle, halogène, alkyle, alkényle, alcoxy, alkylthio, dialkylamino, nitrile, ester, amide, aryl, aryloxy, arylthio, fluoroalkyl, fluoroalcoxy, fluoroalkylthio.

5. Procédé selon la revendication 4 caractérisé en ce que dans la formule générale (I) R est choisi parmi les groupes alkyle ou alkényle linéaire ou ramifié contenant 1 à 12 atomes de carbone.

6. Procédé selon la revendication 1 caractérisé en ce que le solvant aprotique polaire est choisi parmi au moins un des solvants suivants : le diméthylformamide, le diméthylsulfoxide, le diméthylacétamide, la N méthylpyrolidone, l'hexaméthylphosphoramide.

7. Procédé selon la revendication 6 caractérisé en ce que le solvant est choisi parmi le diméthylformamide et/ou les pyridines.

8. Procédé selon la revendication 7 caractérisé en ce que les pyridines sont choisies parmi la pyridine, les alkylpyridines.

9. Procédé selon la revendication 1 caractérisé en ce que le rapport molaire du zinc au dérivé carbonylé est supérieur ou égal à 1 et inférieur à 2.

10. Procédé selon la revendication 1 caractérisé en ce que le rapport molaire de l'iodure ou du bromure de perfluoroalkyle au dérivé carbonylé est supérieur ou égal à 1.

## Claims

1. Process for the perfluoroalkylation of carbonyl compounds, characterized in that a carbonyl-containing derivative, a perfluoroalkyl iodide or bromide and zinc are placed together in a polar aprotic solvent and/or a pyridine in the absence of ultrasonic waves.

2. Process according to Claim 1, characterized in that the perfluoroalkyl bromide is bromotrifluoromethane.

3. Process according to Claim 1, characterized in that the perfluoroalkyl iodide is chosen from perfluoroalkyl iodides, the perfluoroalkyl chain of which contains 2 to 12 carbon atoms.

4. Process according to Claim 1, characterized in that the carbonyl-containing derivative corresponds to the general formula (I):

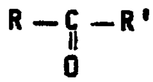

in which
R and R', which may be identical or different, are chosen from hydrogen, straight-chain or branched alkyl or alkenyl groups containing 1 to 20 carbon atoms, alicyclic or mono-, poly-, or heterocyclic aromatic groups optionally substituted by a radical chosen from the constituents: hydroxyl, halogen, alkyl, alkenyl, alkoxy, alkylthio, dialkylamino, nitrile, ester, amide, aryl, aryloxy, arylthio, fluoroalkyl, fluoroalkoxy and fluoroalkylthio.

5. Process according to Claim 4, characterized in that in the general formula (I) R is chosen from straight-chain or branched alkyl or alkenyl groups containing 1 to 12 carbon atoms.

6. Process according to Claim 1, characterized in that the polar aprotic solvent is chosen from at least one of the following solvents: dimethylformamide, dimethyl sulphoxide, dimethylacetamide, N-methylpyrrolidone and hexamethylphosphoramide.

7. Process according to Claim 6, characterized in that the solvent is chosen from dimethylformamide and/or pyridines.

8. Process according to Claim 7, characterized in that the pyridines are chosen from pyridine and alkylpyridines.

9. Process according to Claim 1, characterized in that the molar ratio of zinc to the carbonyl-containing derivative is greater than or equal to 1 and less than 2.

10. Process according to Claim 1, characterized in that the molar ratio of the perfluoroalkyl iodide or bromide to the carbonyl-containing derivative is greater than or equal to 1.

**Patentansprüche**

1. Verfahren zur Perfluoralkylierung von Carbonylverbindungen, dadurch gekennzeichnet, daß man in Gegenwart eines Carbonyl-Derivats ein Perfluoralkyljodid oder -bromid und Zink in einem polaren aprotischen Lösungsmittel und/oder einem Pyridin in Abwesenheit von Ultraschall umsetzt.

2. Verfahren nach Anspruch 1, dadurch gekennzeichnet, daß das Perfluoralkylbromid das Bromtrifluormethan ist.

3. Verfahren nach Anspruch 1, dadurch gekennzeichnet, daß das Perfluoralkyljodid ausgewählt wird aus den Perfluoralkyljodiden, deren perfluorierte Alkylkette 2 bis 12 Kohlenstoffatome enthält.

4. Verfahren nach Anspruch 1, dadurch gekennzeichnet, daß das Carbonylderivat der allgemeinen Formel (I) entspricht

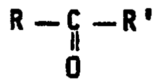

in der
R und R′ identisch oder verschieden sind und ausgewählt werden aus einem Wasserstoffatom, den linearen oder verzweigten Alkyl- oder Alkenylgruppen mit 1 bis 20 Kohlenstoffatomen, alicyclischen Gruppen, monocyclischen, polycyclischen oder heterocyclischen aromatischen Gruppen, die gegebenenfalls mit einem Rest ausgewählt aus den Elementen: Hydroxyl, Halogen, Alkyl, Alkenyl, Alkoxy, Alkylthio, Dialkylamino, Nitril, Ester, Amid, Aryl, Aryloxy, Arylthio, Fluoralkyl, Fluoralkoxy, Fluoralkylthio substituiert sind.

5. Verfahren nach Anspruch 4, dadurch gekennzeichnet, daß in der allgemeinen Formel (I) R ausgewählt wird aus den linearen oder verzweigten Alkylgruppen oder Alkenylgruppen mit 1 bis 12 Kohlenstoffatomen.

6. Verfahren nach Anspruch 1, dadurch gekennzeichnet, daß das polare aprotische Lösungsmittel ausgewählt wird aus wenigstens einem der nachfolgenden Lösungsmittel: Dimethylformamid, Dimethylsulfoxid, Dimethylacetamid, N-Methylpyrrolidon, Hexamethylphosphoramid.

7. Verfahren nach Anspruch 6, dadurch gekennzeichnet, daß das Lösungsmittel ausgewählt wird aus Dimethylformamid und/oder den Pyridinen.

8. Verfahren nach Anspruch 7, dadurch gekennzeichnet, daß die Pyridine ausgewählt werden aus Pyridin und den Alkylpyridinen.

9. Verfahren nach Anspruch 1, dadurch gekennzeichnet, daß das molare Verhältnis von Zink zu Carbonylderivat größer oder gleich 1 und kleiner als 2 ist.

10. Verfahren nach Anspruch 1, dadurch gekennzeichnet, daß das molare Verhältnis des Perfluoralkyljodids oder -bromids zu dem Carbonylderivat größer oder gleich 1 ist.